(19) Europäisches Patentamt
European Patent Office
Office européen des brevets

(11) EP 4 458 946 A1

(12) **EUROPEAN PATENT APPLICATION**
published in accordance with Art. 153(4) EPC

(43) Date of publication:
**06.11.2024 Bulletin 2024/45**

(21) Application number: **22916001.5**

(22) Date of filing: **26.12.2022**

(51) International Patent Classification (IPC):
***C12M 3/00*** *(2006.01)* ***C07K 5/09*** *(2006.01)*
***C07K 5/10*** *(2006.01)* ***C07K 5/11*** *(2006.01)*
***C07K 7/06*** *(2006.01)* ***C07K 17/08*** *(2006.01)*
***C08F 261/12*** *(2006.01)* ***C08G 81/02*** *(2006.01)*
***C08L 101/06*** *(2006.01)* ***C12M 1/00*** *(2006.01)*
***C12N 1/00*** *(2006.01)* ***C12N 5/071*** *(2010.01)*

(52) Cooperative Patent Classification (CPC):
**C07K 5/0815; C07K 5/10; C07K 5/1019; C07K 7/06; C07K 17/08; C08F 261/12; C08G 81/02; C08L 101/06; C12M 1/00; C12M 3/00; C12N 1/00; C12N 5/06**

(86) International application number:
**PCT/JP2022/047836**

(87) International publication number:
**WO 2023/127778 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 JP 2021212493**
**06.01.2022 JP 2022001013**
**06.01.2022 JP 2022001014**
**10.05.2022 JP 2022077686**

(71) Applicant: **SEKISUI CHEMICAL CO., LTD.**
**Osaka-shi**
**Osaka**
**530-8565 (JP)**

(72) Inventors:
- **ARAI, Yuuhei**
  **Mishima-gun, Osaka 618-0021 (JP)**
- **KOBAYASHI, Daigo**
  **Mishima-gun, Osaka 618-0021 (JP)**
- **ARAI, Yoshito**
  **Mishima-gun, Osaka 618-0021 (JP)**

(74) Representative: **Ter Meer Steinmeister & Partner**
**Patentanwälte mbB**
**Nymphenburger Straße 4**
**80335 München (DE)**

Remarks:
The complete document including Reference Table(s) and the Sequence Listing(s) can be downloaded from the EPO website

# (54) SCAFFOLD MATERIAL FOR CELL CULTURE

(57) Provided is a scaffold material for cell culture with which the culture stability of cells can be maintained over an extended period of time. A scaffold material for cell culture according to the present invention contains a peptide-conjugated (meth)acrylic copolymer having a (meth)acrylic copolymer moiety and a peptide moiety bonded to the (meth)acrylic copolymer moiety, when the peptide-conjugated (meth)acrylic copolymer is subjected to high performance liquid chromatography measurement under the following condition 1, a peak top of a main peak having the largest area among signals being not detected within a retention time of 4 minutes:
the condition 1 being:
a column of C18 (Inner diameter 3.0 mm × length 150 mm, filler particle size 3.5 μm),
a column temperature of 40°C,
a flow rate of 0.3 mL/min,
a detector of an evaporative light scattering detector,
a mobile phase A (liquid A) of a 0.1 wt% aqueous formic acid solution,
a mobile phase B (liquid B) of isopropyl alcohol,
a retention time of 0 minutes, in which the liquid A/the liquid B (volume ratio) is 70%/30%,
a retention time of 0 minutes to 15 minutes, in which the liquid A/the liquid B (volume ratio) is changed from 70%/30% to 0%/100%, and

EP 4 458 946 A1

a retention time of 15 minutes to 30 minutes, in which the liquid A/the liquid B (volume ratio) = 0%/100% is maintained

[FIG. 1]

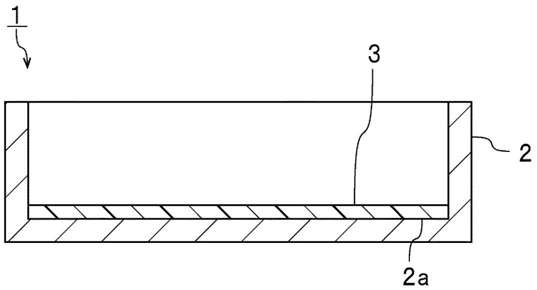

## Description

### TECHNICAL FIELD

The present invention relates to a scaffold material for cell culture.

### BACKGROUND ART

Cells of animals such as humans, mouse, rat, pig, cow, and monkey are used in research and development in academic fields, drug discovery fields, regenerative medicine fields, and the like. As a scaffold material used for culturing animal cells, adhesive proteins such as laminin and vitronectin, and natural polymer materials such as matrigel derived from mouse sarcoma are used.

A scaffold material using a synthetic resin and a scaffold material using a synthetic resin to which a peptide is bonded are also known.

Patent Document 1 below discloses an article for cell culture coated with a composition containing a polymer in which an acrylic polymer and a polypeptide are bonded. In Patent Document 1, a hydrophilic acrylic polymer obtained by polymerizing a hydrophilic acrylic monomer is used as the acrylic polymer.

Patent Document 2 below discloses a coating composition for adhesive cell culture in which a water-insoluble polymer compound is dissolved in a lower alcohol or a mixed solvent of a lower alcohol and water. Patent Document 2 describes, as the water-insoluble polymer compound, a copolymer of a (meth)acrylic acid derivative chemically modified with a peptide and a hydrophilic acrylate compound. As the copolymer, a copolymer having a relatively high content ratio of the hydrophilic acrylate compound is used.

### Related Art Document

### Patent Documents

Patent Document 1: WO 2012/158235 A2
Patent Document 2: JP H5-292957 A

### SUMMARY OF THE INVENTION

### PROBLEMS TO BE SOLVED BY THE INVENTION

As described in Patent Documents 1 and 2, a scaffold material for cell culture using an acrylic copolymer to which a peptide is bonded is known. Cells can be cultured in a liquid medium using a scaffold material, for example, processed or molded into a predetermined shape.

However, in the conventional scaffold materials as described in Patent Documents 1 and 2, since the acrylic copolymer having relatively high hydrophilicity is used, it is easy for the scaffold material to be gradually eluted into the liquid medium during cell culture or for the scaffold material to be detached from a container or the like during cell culture. Therefore, in the conventional scaffold materials as described in Patent Documents 1 and 2, as the number of days of culture of the cells increases, the culture stability of the cells tends to decrease, for example, the proliferation speed of the cells decreases.

An object of the present invention is to provide a scaffold material for cell culture with which the culture stability of cells can be maintained over an extended period of time.

### MEANS FOR SOLVING THE PROBLEMS

A scaffold material for cell culture according to a first invention of the present application contains a peptide-conjugated (meth)acrylic copolymer having a (meth)acrylic copolymer moiety and a peptide moiety bonded to the (meth)acrylic copolymer moiety, when the peptide-conjugated (meth)acrylic copolymer is subjected to high performance liquid chromatography measurement under the following condition 1, a peak top of a main peak having the largest area among signals being not detected within a retention time of 4 minutes.

[Condition 1]

Column: C18 (Inner diameter 3.0 mm × length 150 mm, filler particle size 3.5 μm)
Column temperature: 40°C
Flow rate: 0.3 mL/min
Detector: Evaporative light scattering detector
Mobile phase A (liquid A): 0.1 wt% aqueous formic acid solution
Mobile phase B (liquid B): Isopropyl alcohol
Retention time 0 minutes: Liquid A/the liquid B (volume ratio) = 70%/30%
Retention time 0 minutes to 15 minutes: Liquid A/the liquid B (volume ratio) being changed from 70%/30% to 0%/100%
Retention time 15 minutes to 30 minutes: Liquid A/the liquid B (volume ratio) = 0%/100% being maintained

In a certain aspect of the scaffold material for cell culture according to the first invention of the present application, when the peptide-conjugated (meth)acrylic copolymer is subjected to high performance liquid chromatography measurement under the condition 1, a retention time of a peak top in a main peak having the largest area among signals to be detected is 5 minutes or more and 30 minutes or less.

A scaffold material for cell culture according to a second invention of the present application contains a peptide-conjugated (meth)acrylic copolymer having a (meth)acrylic copolymer moiety and a peptide moiety bonded to the (meth)acrylic copolymer moiety, when the peptide-conjugated (meth)acrylic copolymer is subjected to high performance liquid chromatography measurement under the following condition 2, a retention time of a peak top in a main peak having the largest area among signals to be detected being 5 minutes or more and 30 minutes or less.

[Condition 2]

Column: C18 (Inner diameter 3.0 mm × length 150 mm, filler particle size 3.5 μm)
Column temperature: 40°C
Flow rate: 0.3 mL/min
Detector: Evaporative light scattering detector
Mobile phase A (liquid A): 0.1 wt/vol methanol formate
Mobile phase B (liquid B): THF/isopropyl alcohol = 7/3 (volume ratio)
Retention time 0 minutes to 2 minutes: Liquid A/the liquid B (volume ratio) = 100%/0% being maintained
Retention time 2 minutes to 5 minutes: Liquid A/the liquid B (volume ratio) being changed from 100%/0% to 90%/10%
Retention time 5 minutes to 17 minutes: Liquid A/the liquid B (volume ratio) being changed from 90%/10% to 0%/100%
Retention time 17 minutes to 30 minutes: Liquid A/the liquid B (volume ratio) = 0%/100% being maintained

Hereinafter, the first invention and the second invention of the present application may be collectively referred to as the present invention.

In a certain aspect of the scaffold material for cell culture according to the present invention, the (meth)acrylic copolymer moiety has a structural unit derived from a (meth)acrylate compound (A) represented by the following Formula (A1) or the following Formula (A2).

[Chemical 1]

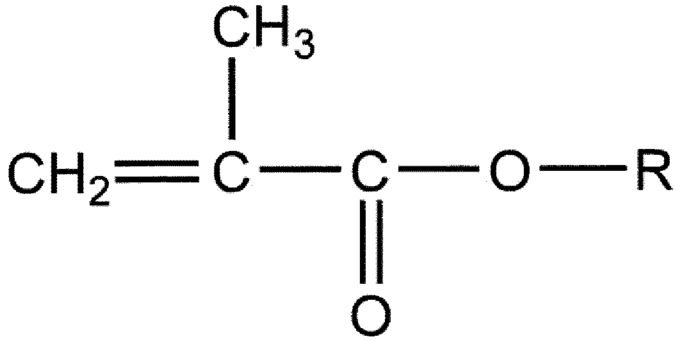 ···(A1)

In the Formula (A1), R represents a hydrocarbon group having 2 or more and 18 or less carbon atoms.

[Chemical 2]

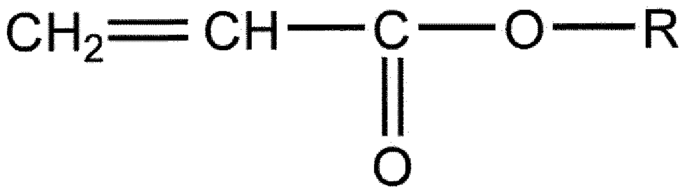 ···(A2)

In the Formula (A2), R represents a hydrocarbon group having 2 or more and 18 or less carbon atoms.

In a certain aspect of the scaffold material for cell culture according to the present invention, the (meth)acrylic copolymer moiety has a structural unit derived from a (meth)acrylate compound (B) having a functional group capable of reacting with an amino group or a carboxyl group, and in the peptide-conjugated (meth)acrylic copolymer, the peptide moiety is bonded to the functional group capable of reacting with an amino group or a carboxyl group.

In a certain aspect of the scaffold material for cell culture according to the present invention, a content ratio of the structural unit derived from the (meth)acrylate compound (A) is 25 mol% or more and 98 mol% or less in 100 mol% of the total structural units of the (meth)acrylic copolymer moiety.

In a certain aspect of the scaffold material for cell culture according to the present invention, a number average molecular weight of the peptide-conjugated (meth)acrylic copolymer is 5000 or more.

In a certain aspect of the scaffold material for cell culture according to the present invention, in the peptide-conjugated (meth)acrylic copolymer, a content ratio of the peptide moiety is 0.5 mol% or more and 25 mol% or less.

In a certain aspect of the scaffold material for cell culture according to the present invention, the peptide moiety has an RGD sequence.

## EFFECT OF THE INVENTION

According to the present invention, it is possible to provide a scaffold material for cell culture with which the culture stability of cells can be maintained over an extended period of time.

## BRIEF DESCRIPTION OF DRAWINGS

[Fig. 1] Fig. 1 is a cross-sectional view schematically illustrating a cell culture container according to an embodiment of the present invention.

[Fig. 2] Fig. 2 is a cross-sectional view schematically illustrating a cell culture microcarrier according to an embodiment of the present invention.

[Fig. 3] Fig. 3 is phase-contrast micrographs at 24 hours, 48 hours, and 72 hours after seeding of cells when cell culture is performed using scaffold materials for cell culture obtained in Examples 1 and 8 and Comparative Example 2.

## MODES FOR CARRYING OUT THE INVENTION

Hereinafter, details of the present invention will be described. In the present specification, the "scaffold material for cell culture" may be abbreviated as "scaffold material".

The scaffold material according to the first invention of the present application contains a peptide-conjugated (meth)acrylic copolymer having a (meth)acrylic copolymer moiety and a peptide moiety bonded to the (meth)acrylic copolymer moiety. In the scaffold material according to the first invention of the present application, when the peptide-conjugated (meth)acrylic copolymer is subjected to high performance liquid chromatography measurement under the following condition 1, a peak top of a main peak having the largest area among signals is not detected within a retention time of 4 minutes.

[Condition 1]

Column: C18 (Inner diameter 3.0 mm × length 150 mm, filler particle size 3.5 μm)
Column temperature: 40°C
Flow rate: 0.3 mL/min
Detector: evaporative light scattering detector
Mobile phase A (liquid A): 0.1 wt% aqueous formic acid solution
Mobile phase B (liquid B): isopropyl alcohol
Retention time of 0 minutes: the liquid A/the liquid B (volume ratio) is 70%/30%
Retention time of 0 minutes to 15 minutes: the liquid A/the liquid B (volume ratio) is changed from 70%/30% to 0%/100%
Retention time of 15 minutes to 30 minutes: the liquid A/the liquid B (volume ratio) = 0%/100% is maintained

Specifically, the high performance liquid chromatography measurement (hereinafter, the "high performance liquid chromatography" may be abbreviated as "HPLC") under the condition 1 can be performed, for example, as follows.

First, a measurement sample can be obtained by adjusting the concentration of the peptide-conjugated (meth)acrylic copolymer to 2 mg/mL using a mixed solution of a 0.1 wt% aqueous formic acid solution and isopropyl alcohol (0.1 wt% aqueous formic acid solution/isopropyl alcohol = 7/3 [volume ratio]) as a solvent.

When the scaffold material contains a component other than the peptide-conjugated (meth)acrylic copolymer, the peptide-conjugated (meth)acrylic copolymer is isolated and used as a measurement sample. When the scaffold material is coated on a culture container or the like, the scaffold material coated on the culture container or the like is immersed in ethanol, and the peptide-conjugated (meth)acrylic copolymer dissolved in ethanol is used as a measurement sample.

As a measuring instrument, for example, an HPLC apparatus (High pressure gradient HPLC system Prominence manufactured by SHIMADZU CORPORATION), an HPLC column (XBridge (registered trademark) C18 [inner diameter 3.0 mm × length 150 mm, filler particle size 3.5 pm] manufactured by Waters Corporation), and an evaporative light scattering detector (ELSD_LTII manufactured by SHIMADZU CORPORATION) can be used. The analysis can be performed, for example, in the following procedure.

A 0.1 wt% aqueous formic acid solution is used as the mobile phase A (liquid A), and isopropyl alcohol is used as the mobile phase B (liquid B). At the start of the measurement, the inside of the HPLC apparatus is filled with a mixed solvent in which the mobile phase A/the mobile phase B is 7/3 in terms of volume ratio. In this state, the measurement sample (injection amount: 20 μL) is injected. Over 15 minutes immediately after the sample injection, the ratio of the mobile phase B is increased at a constant speed so that the ratio of the mobile phase B in the mobile phases is 100% in terms of volume ratio. After 15 minutes (at this point, the mobile phase is completely replaced by the mobile phase B), the mobile phase B is caused to flow for 15 minutes. The column temperature is set to 40°C, and the liquid delivery flow rate is set to a total flow rate of 0.3 mL/min.

As a nebulizer gas of the evaporative light scattering detector, nitrogen gas is used. The gas supply pressure is set to 350 kPa, and the drift tube temperature is set to 40°C. The determination of the baseline can be performed by analyzing a blank test solution prepared in the same manner as in the preparation of the analysis sample except that the scaffold material is dissolved.

The scaffold material according to the second invention of the present application contains a peptide-conjugated (meth)acrylic copolymer having a (meth)acrylic copolymer moiety and a peptide moiety bonded to the (meth)acrylic copolymer moiety. In the scaffold material according to the second invention of the present application, when the peptide-conjugated (meth)acrylic copolymer is subjected to high performance liquid chromatography measurement under the following condition 2, a retention time of a peak top in a main peak having the largest area among signals to be detected

is 5 minutes or more and 30 minutes or less.

[Condition 2]

Column: C18 (Inner diameter 3.0 mm × length 150 mm, filler particle size 3.5 μm)
Column temperature: 40°C
Flow rate: 0.3 mL/min
Detector: evaporative light scattering detector
Mobile phase A (liquid A): 0.1 wt/vol methanol formate
Mobile phase B (liquid B): THF/isopropyl alcohol = 7/3 (volume ratio)
Retention time of 0 minutes to 2 minutes: the liquid A/the liquid B (volume ratio) = 100%/0% is maintained
Retention time of 2 minutes to 5 minutes: the liquid A/the liquid B (volume ratio) is changed from 100%/0% to 90%/10%
Retention time of 5 minutes to 17 minutes: the liquid A/the liquid B (volume ratio) is changed from 90%/10% to 0%/100%
Retention time of 17 minutes to 30 minutes: the liquid A/the liquid B (volume ratio) = 0%/100% is maintained

Specifically, the high performance liquid chromatography measurement (HPLC measurement) under the condition 2 can be performed in the same manner as in the condition 1 except for the following points.

A measurement sample can be obtained by adjusting the concentration of the scaffold material to 2 mg/mL using a mixed solution of tetrahydrofuran (THF) and isopropyl alcohol (THF : IPA = 7 : 3 [volume ratio]) as a solvent. The analysis can be performed, for example, in the following procedure.

As the mobile phase A (liquid A), 0.1 wt/vol methanol formate is used, and as the mobile phase B (liquid B), a mixed solution of THF and IPA (THF : IPA = 7 : 3 [volume ratio]) is used. At the start of the measurement, the volume ratio of the mobile phase A is set to 100%, and the mobile phase A is caused to flow at a constant volume ratio of 100% for 0 minutes to 2 minutes. From 2 minutes to 5 minutes, the ratio of the mobile phase B is increased at a constant speed so that the mobile phase A/the mobile phase B is 90%/10% in terms of volume ratio at the time point of 5 minutes. From 5 minutes to 17 minutes, the ratio of the mobile phase B is increased at a constant speed so that the mobile phase B is 100% in terms of volume ratio at the time point of 17 minutes. From 17 minutes to 30 minutes, the mobile phase B is caused to flow at a constant volume ratio of 100%. The other points are the same as those in the condition 1.

Each of the first invention and the second invention of the present application may be implemented alone, or in combination as described above. Hereinafter, the first invention and the second invention may be collectively referred to as the present invention.

Since the scaffold material according to the present invention is provided with the above configuration, the culture stability of cells can be maintained over an extended period of time.

In the scaffold material according to the present invention, since the hydrophobicity is relatively large and the hydrophobicity is appropriately adjusted by HPLC measurement, the scaffold material is less likely to be eluted into a liquid medium during cell culture, and the scaffold material is less likely to be detached from a container or the like during cell culture. Therefore, in the scaffold material according to the present invention, even when cells are cultured for a long period of time, the proliferation speed of the cells is less likely to decrease.

In the scaffold material according to the present invention, solubility in an alcohol solvent such as ethanol can be improved. Therefore, for example, by applying a coating solution obtained by dissolving the scaffold material in ethanol to a surface of a container or the like and then volatilizing the ethanol, a scaffold material layer having a predetermined shape can be formed on the surface of the container or the like. When the solubility in an alcohol solvent is favorable, the concentration of the scaffold material in the coating solution can be increased, so that a scaffold material layer having a large thickness can be formed.

In the scaffold material according to the present invention, since it is not necessary to use a natural polymer material such as an extracellular matrix (ECM) as a material, it is inexpensive, has little variation between lots, and is excellent in safety.

In the scaffold material, when the peptide-conjugated (meth)acrylic copolymer is subjected to HPLC measurement under the above condition 1, a retention time of a peak top in a main peak having the largest area among signals to be detected is preferably more than 4 minutes, more preferably 5 minutes or more, still more preferably 10 minutes or more, further preferably 15 minutes or more, and particularly preferably 24 minutes or more. When the retention time of the peak top is the lower limit or more, the hydrophobicity of the peptide-conjugated (meth)acrylic copolymer can be further increased, and the effects of the present invention can be more effectively exhibited. The retention time of the peak top may be within 30 minutes, but may be more than 30 minutes. When the main peak is not detected even if the retention time exceeds 30 minutes, the retention time of the peak top exceeds 30 minutes. In the first invention of the

present application, the retention time of the peak top exceeds 4 minutes.

In the scaffold material, when the peptide-conjugated (meth)acrylic copolymer is subjected to HPLC measurement under the above condition 2, a retention time of a peak top in a main peak having the largest area among signals to be detected is preferably 2 minutes or more, more preferably 5 minutes or more, still more preferably 8 minutes or more, further preferably 10 minutes or more, still further preferably 15 minutes or more, and particularly preferably 18 minutes or more, and is preferably 30 minutes or less, more preferably 28 minutes or less, still more preferably 27 minutes or less, further preferably 26 minutes or less, and most preferably 25 minutes or less. When the retention time of the peak top is the lower limit or more, the hydrophobicity of the peptide-conjugated (meth)acrylic copolymer can be further increased, and the effects of the present invention can be more effectively exhibited. When the retention time of the peak top is the upper limit or less, the solubility in an alcohol solvent such as ethanol can be further improved, and thus coatability and processability can be further improved. In the second invention of the present application, the retention time of the peak top is 5 minutes or more and 30 minutes or less.

When the scaffold material is subjected to HPLC measurement under the above condition 1 or 2, the retention time of the peak top in the main peak having the largest area among signals to be detected can be adjusted, for example, by the structure, molecular weight, and the like of the peptide-conjugated (meth)acrylic copolymer. Specifically, as described below, the retention time can be lengthened, for example, by increasing the ratio of the hydrophobic (meth)acrylate compound in the peptide-conjugated (meth)acrylic copolymer or moderately increasing the molecular weight of the peptide-conjugated (meth)acrylic copolymer.

Hereinafter, details of the scaffold material will be further described. In the present specification, "(meth)acrylic" means one or both of "acrylic" and "methacrylic", and "(meth)acrylate" means one or both of "acrylate" and "methacrylate".

(Peptide-conjugated (meth)acrylic copolymer)

The scaffold material contains a peptide-conjugated (meth)acrylic copolymer. The peptide-conjugated (meth)acrylic copolymer is a (meth)acrylic copolymer to which a peptide is bonded. The peptide-conjugated (meth) acrylic copolymer has a (meth) acrylic copolymer moiety and a peptide moiety bonded to the (meth)acrylic copolymer moiety. Only one kind of the peptide-conjugated (meth) acrylic copolymer may be used, or two or more kinds thereof may be used in combination.

<(Meth)acrylic copolymer moiety>

The (meth)acrylic copolymer moiety preferably has a structural unit derived from a (meth)acrylate compound (A) represented by the following Formula (A1) or the following Formula (A2) . Thereby, the hydrophobicity of the peptide-conjugated (meth) acrylic copolymer can be further increased. When the scaffold material is subjected to HPLC measurement under the above condition 1 or 2, the retention time of the peak top in the main peak having the largest area among signals to be detected can be increased. The (meth)acrylate compound (A) may contain a (meth)acrylate compound represented by the following Formula (A1), may contain a (meth)acrylate compound represented by the following Formula (A2), or may contain both a (meth)acrylate compound represented by the following Formula (A1) and a (meth)acrylate compound represented by the following Formula (A2). When the (meth)acrylate compound (A) contains both a (meth)acrylate compound represented by the following Formula (A1) and a (meth)acrylate compound represented by the following Formula (A2), R in the following Formula (A1) and R in the following Formula (A2) may be the same or different. Only one kind of the (meth)acrylate compound (A) may be used, or two or more kinds thereof may be used in combination. Only one kind of each of the (meth)acrylate compound represented by the following Formula (A1) and the (meth)acrylate compound represented by the following Formula (A2) may be used, or two or more kinds thereof may be used in combination.

[Chemical 3]

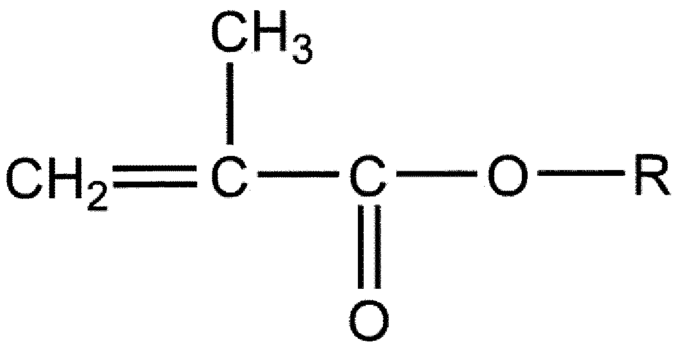

···(A1)

In the above Formula (A1), R represents a hydrocarbon group having 2 or more and 18 or less carbon atoms.

[Chemical 4]

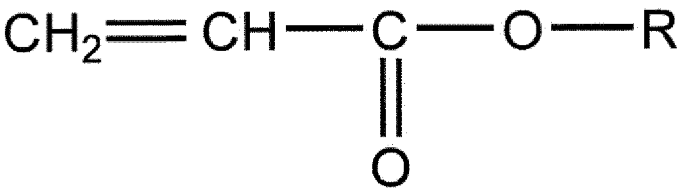

···(A2)

In the above Formula (A2), R represents a hydrocarbon group having 2 or more and 18 or less carbon atoms.

Each of R in the above Formula (A1) and R in the above Formula (A2) may be an aliphatic hydrocarbon group or an aromatic hydrocarbon group. From the viewpoint of further enhancing the solubility of the peptide-conjugated (meth)acrylic copolymer, each of R in the above Formula (A1) and R in the above Formula (A2) is preferably an aliphatic hydrocarbon group. The aliphatic hydrocarbon group may be linear, may have a branched structure, may have a double bond, or may not have a double bond. Each of R in the above Formula (A1) and R in the above Formula (A2) may be an alkyl group or an alkylene group.

The number of carbon atoms of R in the above Formula (A1) and the number of carbon atoms of R in the above Formula (A2) are each preferably 4 or more, more preferably 6 or more, still more preferably 8 or more, and particularly preferably 10 or more, and is preferably 16 or less, more preferably 14 or less, and most preferably 12. When the number of carbon atoms is the lower limit or more, the hydrophobicity of the peptide-conjugated (meth)acrylic copolymer can be further increased. When the peptide-conjugated (meth)acrylic copolymer is subjected to HPLC measurement under the above condition 1 or 2, the retention time of the peak top in the main peak having the largest area among signals to be detected can be further increased. If the number of carbon atoms is the upper limit or less, when the peptide-conjugated (meth)acrylic copolymer is subjected to HPLC measurement under the above condition 1 or 2, the retention time of the peak top in the main peak having the largest area among signals to be detected can be further decreased. Since the solubility of the peptide-conjugated (meth)acrylic copolymer in an alcohol solvent such as ethanol can be further improved, coatability and processability can be further improved. In particular, when the number of carbon atoms is 12, the effects of the present invention can be further more effectively exhibited, and coatability and processability can be further enhanced.

The content ratio of the structural unit derived from the (meth)acrylate compound (A) in 100 mol% of the total structural units of the (meth)acrylic copolymer moiety is preferably 25 mol% or more, more preferably 30 mol% or more, still more preferably 40 mol% or more, and particularly preferably 50 mol% or more, and is preferably 98 mol% or less, more preferably 95 mol% or less, still more preferably 90 mol% or less, and particularly preferably 80 mol% or less. The content ratio of the structural unit derived from the (meth)acrylate compound (A) in 100 mol% of the total structural units of the (meth)acrylic copolymer moiety is preferably 25 mol% or more and 98 mol% or less, more preferably 30 mol% or more and 95 mol% or less, still more preferably 40 mol% or more and 90 mol% or less, and particularly preferably 50 mol% or more and 80 mol% or less. When the content ratio is the lower limit or more, the hydrophobicity of the peptide-conjugated (meth)acrylic copolymer can be further increased. When the peptide-conjugated (meth)acrylic copolymer is subjected to HPLC measurement under the above condition 1 or 2, the retention time of the peak top in the main peak having the largest area among signals to be detected can be further increased. If the content ratio is the upper limit or less, when the peptide-conjugated (meth)acrylic copolymer is subjected to HPLC measurement under the above condition

1 or 2, the retention time of the peak top in the main peak having the largest area among signals to be detected can be further decreased. Since the solubility of the peptide-conjugated (meth)acrylic copolymer in an alcohol solvent such as ethanol can be further improved, coatability and processability can be further improved.

The (meth)acrylic copolymer moiety preferably has a structural unit derived from a (meth)acrylate compound (B) having a functional group capable of reacting with an amino group or a carboxyl group. The (meth)acrylate compound (B) may have a functional group capable of reacting with an amino group, may have a functional group capable of reacting with a carboxyl group, and may have a functional group capable of reacting with an amino group and a functional group capable of reacting with a carboxyl group. Only one kind of the (meth) acrylate compound (B) may be used, or two or more kinds thereof may be used in combination.

Examples of the functional group capable of reacting with an amino group or a carboxyl group include a carboxyl group, a thiol group, an amino group, and a cyano group.

From the viewpoint of effectively exhibiting the effects of the present invention, in the peptide-conjugated (meth)acrylic copolymer, the peptide moiety is preferably bonded to the functional group capable of reacting with an amino group or a carboxyl group. More specifically, the carboxyl group or amino group of the amino acid constituting the peptide moiety is preferably bonded to the functional group capable of reacting with an amino group or a carboxyl group.

The functional group capable of reacting with an amino group or a carboxyl group is preferably a carboxyl group or an amino group. The (meth)acrylate compound (B) preferably has a carboxyl group or an amino group.

Examples of the (meth)acrylate compound (B) include (meth)acrylic acid, 3-butenoic acid, 4-pentenoic acid, 5-hexenoic acid, 6-heptenoic acid, 7-octenoic acid, benzene acrylic acid, (meth)acryloyloxyethylsuccinic acid, (meth)acryloyloxyethylphthalic acid, (meth)acryloyloxypropylsuccinic acid, (meth)acryloyloxypropylphthalic acid, (meth)acryloyloxyethylhexahydrosuccinic acid, (meth)acryloyloxyethylhexahydrophthalic acid, (meth)acryloyloxypropylhexahydrosuccinic acid, and (meth)acryloyloxypropylhexahydrophthalic acid.

The (meth)acrylate compound (B) is preferably (meth)acrylic acid, (meth)acryloyloxyethylsuccinic acid, (meth)acryloyloxypropylsuccinic acid, (meth)acryloyloxyethylhexahydrosuccinic acid, (meth)acryloyloxypropylhexahydrosuccinic acid, or butenoic acid, and more preferably (meth)acrylic acid. In this case, the effects of the present invention can be more effectively exhibited.

The content ratio of the structural unit derived from the (meth) acrylate compound (B) in 100 mol% of the total structural units of the (meth)acrylic copolymer moiety is preferably 2 mol% or more, more preferably 5 mol% or more, and still more preferably 10 mol% or more, and is preferably 75 mol% or less, more preferably 70 mol% or less, and still more preferably 60 mol% or less. When the content ratio is the lower limit or more, the solubility of the peptide-conjugated (meth)acrylic copolymer in an alcohol solvent can be further enhanced. When the content ratio is the upper limit or less, the culture stability of cells is more easily maintained over an extended period of time.

The total content ratio of the structural unit derived from the (meth)acrylate compound (A) and the structural unit derived from the (meth)acrylate compound (B) in 100 mol% of the total structural units of the (meth)acrylic copolymer moiety is preferably 50 mol% or more, more preferably 65 mol% or more, still more preferably 80 mol% or more, still further preferably 90 mol% or more, particularly preferably 95 mol% or more, and most preferably 100 mol%. When the total content ratio is the lower limit or more, the effects of the present invention can be more effectively exhibited. The total content ratio may be 100 mol% or less or 90 mol% or less.

The (meth)acrylic copolymer moiety may include a structural unit derived from a (meth)acrylate compound different from both the (meth)acrylate compound (A) and the (meth)acrylate compound (B) as long as it is not contrary to the object of the present invention. The (meth)acrylic copolymer moiety may include a structural unit derived from a vinyl compound copolymerizable with the (meth)acrylate compound as long as it is not contrary to the object of the present invention.

The content ratio of the structural unit derived from the (meth)acrylate compound (A) and the content ratio of the structural unit derived from the (meth)acrylate compound (B) in the (meth)acrylic copolymer moiety can be measured by, for example, nuclear magnetic resonance (NMR).

<Peptide moiety>

The peptide moiety is a structural moiety derived from a peptide. The peptide moiety has an amino acid sequence. The peptide constituting the peptide moiety may be an oligopeptide or a polypeptide. Only one kind of the peptide may be used, or two or more kinds thereof may be used in combination.

The number of amino acid residues in the peptide moiety is preferably 3 or more, more preferably 4 or more, and still more preferably 5 or more, and is preferably 10 or less, more preferably 8 or less, and still more preferably 6 or less. When the number of amino acid residues is the lower limit or more and the upper limit or less, the adhesion to cells after seeding can be further enhanced, and the proliferation rate of cells can be further enhanced. However, the number of amino acid residues in the peptide moiety may be more than 10 or more than 15.

The peptide moiety preferably has a cell adhesive amino acid sequence. The cell adhesive amino acid sequence

refers to an amino acid sequence whose cell adhesion activity has been confirmed by a phage display method, a sepharose bead method, or a plate coating method. As the phage display method, for example, the method described in "The Journal of Cell Biology, Volume 130, Number 5, September 1995 1189-1196" can be used. As the sepharose bead method, for example, the method described in "PROTEIN, NUCLEIC ACID, AND ENZYME, Vol. 45, No. 15 (2000) 2477" can be used. As the plate coating method, for example, the method described in "PROTEIN, NUCLEIC ACID, AND ENZYME, Vol. 45, No. 15 (2000) 2477" can be used.

Examples of the cell adhesive amino acid sequence include an RGD sequence (Arg-Gly-Asp), a YIGSR sequence (Tyr-Ile-Gly-Ser-Arg), a PDSGR sequence (Pro-Asp-Ser-Gly-Arg), an HAV sequence (His-Ala-Val), an ADT sequence (Ala-Asp-Thr), a QAV sequence (Gln-Ala-Val), an LDV sequence (Leu-Asp-Val), an IDS sequence (Ile-Asp-Ser), an REDV sequence (Arg-Glu-Asp-Val), an IDAPS sequence (Ile-Asp-Ala-Pro-Ser), a KQAGDV sequence (Lys-Gln-Ala-Gly-Asp-Val), and a TDE sequence (Thr-Asp-Glu). Examples of the cell adhesive amino acid sequence include the sequences described in "Pathophysiology, Vol. 9, No. 7, p. 527 to 535, 1990" and "Osaka Medical Center and Research Institute for Maternal and Child Health magazine, Vol. 8, No. 1, p. 58 to 66, 1992". The peptide moiety may have only one type of the cell adhesive amino acid sequence or two or more types thereof.

The cell adhesive amino acid sequence preferably has at least one of the above-described cell adhesive amino acid sequences, more preferably has at least an RGD sequence, a YIGSR sequence, or a PDSGR sequence, still more preferably has an RGD sequence, and particularly preferably has at least an RGD sequence represented by the following Formula (1) . In this case, the adhesion to cells after seeding can be further enhanced, and the proliferation rate of cells can be further enhanced.

Arg-Gly-Asp-X     Formula (1)

In the above Formula (1), X represents Gly, Ala, Val, Ser, Thr, Phe, Met, Pro, or Asn.

The peptide moiety may be linear or may have a cyclic peptide skeleton. The cyclic peptide skeleton is a cyclic skeleton composed of a plurality of amino acids. From the viewpoint of further effectively exhibiting the effects of the present invention, the cyclic peptide skeleton is preferably composed of four or more amino acids, preferably composed of five or more amino acids, and preferably composed of ten or less amino acids.

In the peptide-conjugated (meth)acrylic copolymer, the content ratio of the peptide moiety is preferably 0.5 mol% or more, more preferably 1 mol% or more, and still more preferably 5 mol% or more, and is preferably 25 mol% or less, more preferably 20 mol% or less, and still more preferably 15 mol% or less. When the content ratio of the peptide moiety is the lower limit or more, the adhesion to cells after seeding can be further enhanced, and the proliferation rate of cells can be further enhanced. When the content ratio of the peptide moiety is the upper limit or less, the culture stability of cells can be further enhanced, and the production cost can be suppressed. The content ratio (mol%) of the peptide moiety is the amount of substance of the peptide moiety with respect to the total of the amount of substance of structural units constituting the peptide-conjugated (meth)acrylic copolymer.

The content ratio of the peptide moiety can be measured by, for example, nuclear magnetic resonance (NMR).

<Other details of peptide-conjugated (meth)acrylic copolymer>

The number average molecular weight of the peptide-conjugated (meth)acrylic copolymer is preferably 5000 or more, more preferably 10000 or more, and still more preferably 50000 or more, and is preferably 5000000 or less, more preferably 2500000 or less, and still more preferably 1000000 or less. When the number average molecular weight is the lower limit or more, the solubility of the peptide-conjugated (meth)acrylic copolymer in water can be further decreased, and the effects of the present invention can be more effectively exhibited. When the number average molecular weight is the upper limit or less, the solubility in an alcohol solvent can be further enhanced.

The number average molecular weight of the peptide-conjugated (meth)acrylic copolymer can be measured by, for example, the following method. The peptide-conjugated (meth)acrylic copolymer is dissolved in tetrahydrofuran (THF) to prepare a 0.2 wt% solution of the peptide-conjugated (meth)acrylic copolymer. Next, evaluation is performed under the following measurement conditions using a gel permeation chromatography (GPC) measuring device (APC system, manufactured by Waters Corporation).

Column: HSPgel HR MB-M 6.0 mm × 150 mm
Flow rate: 0.5 mL/min
Column temperature: 40°C
Injection volume: 10 μL
Detector: RI, PDA
Standard sample: Polystyrene

A method for producing the peptide-conjugated (meth)acrylic copolymer is not particularly limited. The peptide-conjugated (meth)acrylic copolymer can be prepared, for example, as follows.

A monomer mixture containing a (meth)acrylate compound (A) and a (meth) acrylate compound (B) is polymerized to obtain a (meth)acrylate copolymer. The obtained (meth)acrylate copolymer is reacted with a peptide to obtain a peptide-conjugated (meth)acrylic copolymer.

(Other details of scaffold material for cell culture)

The scaffold material is used for culturing cells. The scaffold material is used as a scaffold for cells when the cells are cultured.

Examples of the cells include cells of animals such as humans, mouse, rat, pig, cow, and monkey. Examples of the cells include somatic cells, and examples thereof include stem cells, progenitor cells, and mature cells. The somatic cells may be cancer cells.

Examples of the stem cells include somatic stem cells and embryonic stem cells, and examples thereof include neural stem cells, hematopoietic stem cells, mesenchymal stem cells (MSC), iPS cells, ES cells, Muse cells, embryonic cancer cells, embryonic germ stem cells, and mGS cells.

Examples of the mature cells include nerve cells, cardiomyocytes, retinal cells, and hepatocytes.

The scaffold material is preferably used for two-dimensional culture (plane culture), three-dimensional culture, or suspension culture of cells, more preferably used for two-dimensional culture (plane culture) or three-dimensional culture, and still more preferably used for two-dimensional culture.

The scaffold material is preferably used for serum-free medium culture. Since the scaffold material contains the peptide-conjugated (meth)acrylic copolymer, the adhesiveness of cells can be enhanced even in a serum-free medium culture containing no feeder cell or adhesive protein, and in particular, initial fixation rate after cell seeding can be enhanced. Since the scaffold material contains the peptide-conjugated (meth)acrylic copolymer, the effects of the present invention can be exhibited even in a serum-free medium culture.

The content of the peptide-conjugated (meth)acrylic copolymer in 100 wt% of the scaffold material is preferably 90 wt% or more, more preferably 95 wt% or more, still more preferably 97.5 wt% or more, particularly preferably 99 wt% or more, and most preferably 100 wt% (whole amount). When the content of the peptide-conjugated (meth)acrylic copolymer is the lower limit or more, the effects of the present invention can be more effectively exhibited. However, the content of the peptide-conjugated (meth)acrylic copolymer in 100 wt% of the scaffold material may be 100 wt% or less or 98 wt% or less.

The scaffold material may contain components other than the peptide-conjugated (meth)acrylic copolymer. Examples of the components other than the peptide-conjugated (meth)acrylic copolymer include polyolefin resins, polyether resins, polyvinyl alcohol resins, polyesters, epoxy resins, polyamide resins, polyimide resins, polyurethane resins, polycarbonate resins, polysaccharides, celluloses, polypeptides, and synthetic peptides.

It is preferable that the scaffold material does not substantially contain animal-derived raw materials. Since the scaffold material does not contain animal-derived raw materials, it is possible to provide a scaffold material having high safety and little variation in quality during production. The phrase "does not substantially contain animal-derived raw materials" means that the animal-derived raw materials in the scaffold material are 3 wt% or less. In the scaffold material, the animal-derived raw materials in the scaffold material are preferably 1 wt% or less and most preferably 0 wt%. That is, it is most preferable that the scaffold material does not contain any animal-derived raw materials.

The shape of the scaffold material is not particularly limited. The shape of the scaffold material may be a particulate shape, a fibrous shape, a porous shape, or a membrane shape.

The scaffold material is preferably a resin film. The resin film is preferably a resin film formed of the scaffold material. The resin film is a membrane-like scaffold material.

The thickness of the resin film is not particularly limited. The average thickness of the resin film may be 10 nm or more, 50 nm or more, or 500 nm or more, and may be 1000 μm or less or 500 μm or less.

(Cell culture container)

A cell culture container preferably includes the above-described resin film in at least a part of a cell culture area. The cell culture container includes a container body and the above-described resin film, and the resin film is preferably disposed on a surface of the container body. The resin film is preferably a membrane-like scaffold material, and is preferably a scaffold material layer.

Fig. 1 is a cross-sectional view schematically illustrating a cell culture container according to an embodiment of the present invention.

A cell culture container 1 includes a container body 2 and a resin film 3. The resin film 3 is disposed on a surface 2a of the container body 2. The resin film 3 is disposed on a bottom surface of the container body 2. Cells can be cultured

in plane by adding a liquid medium is added to the cell culture container 1 and seeding cells such as cell mass on the surface of the resin film 3.

The container body may include a first container body and a second container body such as a cover glass on a bottom surface of the first container body. The first container body and the second container body may be separable. In this case, the resin film 3 may be disposed on a surface of the second container body.

As the container body, a conventionally known container body (container) can be used. The shape and size of the container body are not particularly limited. As the container body, for example, a 2 to 384-well plate, a single-layer flask, a multi-layer flask, a multi-plane flask, a dish, a roller bottle, a bag, an insert cup, a microchannel chip, or the like can be used.

The material of the container body is not particularly limited, and examples thereof include synthetic resins, metals, and glass. Examples of the synthetic resin include polystyrene, polyethylene, polypropylene, polyethersulfone, polycarbonate, polyester, polyisoprene, a cycloolefin polymer, polyimide, polyamide, polyamideimide, a (meth)acrylic resin, an epoxy resin, and silicone.

(Cell culture microcarrier)

A cell culture microcarrier (hereinafter, may be abbreviated as "microcarrier") includes a base material particle and a coating layer covering an outer surface of the base material particle, and the coating layer is preferably formed of the scaffold material. The coating layer is preferably a scaffold material layer. As the base material particle, a conventionally known base material particle used in a microcarrier can be used. Examples of the base material particle include resin particles.

Fig. 2 is a cross-sectional view schematically illustrating a cell culture microcarrier according to an embodiment of the present invention.

A cell culture microcarrier 5 illustrated in Fig. 2 includes a base material particle 6 and a coating layer 7 covering an outer surface of the base material particle 6. The coating layer 7 is disposed on the surface of the base material particle 6 and is in contact with the surface of the base material particle 6. The coating layer 7 covers the entire outer surface of the base material particle 6.

(Method for culturing cells)

Cells can be cultured using the scaffold material, the resin film, and the microcarrier. The method for culturing cells is a method for culturing cells using the scaffold material. The method for culturing cells is preferably a method for culturing cells using the resin film, and is preferably a method for culturing cells using the microcarrier. Examples of the cells include the above-described cells.

The method for culturing cells preferably includes a step of seeding cells on the scaffold material. The method for culturing cells preferably includes a step of seeding cells on the resin film. The method for culturing cells preferably includes a step of seeding cells on the microcarrier. The cells may be a cell mass. The cell mass can be obtained by adding a cell detachment agent to a confluent culture container and uniformly crushing it by pipetting. The cell detachment agent is not particularly limited, but an ethylenediamine/phosphate buffer solution is preferable. The size of the cell mass is preferably 50 um to 200 $\mu$m.

Hereinafter, the present invention will be described in more detail with reference to Examples and Comparative Examples. The present invention is not limited only to these Examples.

The content ratio of the structural unit and the content ratio of the peptide moiety in the obtained peptide-conjugated (meth)acrylic copolymer were measured by 1H-NMR (nuclear magnetic resonance spectrum) after dissolving the copolymer in DMSO-d6 (dimethyl sulfoxide).

(Example 1)

Preparation of peptide-conjugated (meth)acrylic copolymer:

Butyl acrylate (4.5 parts by weight) and acrylic acid (7.0 parts by weight) were dissolved in 27 parts by weight of tetrahydrofuran to obtain an acrylic monomer solution. In the obtained acrylic monomer solution, 0.0575 parts by weight of Irgacure 184 (manufactured by BASF) was dissolved, and the obtained solution was applied onto a PET film. A (meth) acrylic copolymer solution was obtained by irradiating the coated material with light having a wavelength of 365 nm at an integrated light quantity of 2000 mJ/cm$^2$ using a UV conveyor device ("ECS301G1" manufactured by Eye Graphics Co., Ltd.) at 25°C. The obtained (meth)acrylic copolymer solution was vacuum-dried at 80°C for 3 hours to obtain a (meth)acrylic copolymer.

As the peptide, a cyclic peptide having an amino acid sequence of Arg-Gly-Asp-Phe-Lys (five amino acid

residues, forming a cyclic skeleton by bonding Arg and Lys, Phe is a D form, described as c-RGDfK in the table) was prepared. As a condensing agent, 1-ethyl-3-(3-dimethylaminopropyl)carbodiimide hydrochloride was prepared. A (meth)acrylic copolymer (50 parts by weight) and a peptide (10 parts by weight) were dissolved in 1000 parts by weight of DMF (N,N-dimethylformamide) to prepare a first solution. A condensing agent (1 part by weight) was mixed with 1000 parts by weight of DMF to prepare a second solution. The first solution and the second solution were mixed to prepare a solution containing the (meth)acrylic copolymer, the peptide, and the condensing agent. The obtained solution was reacted at 40°C for 2 hours, and a carboxyl group in the structural unit derived from acrylic acid of the (meth)acrylic copolymer and an amino group of Lys of the peptide were dehydrated and condensed, thereby obtaining a solution containing a peptide-conjugated (meth)acrylic copolymer.

Preparation of coating solution:

The obtained solution containing a peptide-conjugated (meth)acrylic copolymer was diluted 100 times with DMF, and added dropwise to a column packed with an ion exchange resin (manufactured by Organo Corporation) at a rate of 0.3 mL/min for washing. The solution after washing was vacuum-dried at 60°C for 3 hours to obtain a dried solid, and the dried solid was dissolved in ethanol to obtain a coating solution containing a peptide-conjugated (meth)acrylic copolymer and ethanol. The content of the peptide-conjugated (meth)acrylic copolymer in the coating solution was set to 0.1 wt%.

Preparation of cell culture container:

To each well of a 6-well plate, 20 uL of the obtained coating solution was applied by a cast coating method, and then ethanol was removed by vacuum drying at 60°C for 3 hours. In this way, a cell culture container in which a resin film (membrane-like scaffold material) formed of the scaffold material was disposed on the bottom surface of each well was obtained.

(Examples 2 to 10 and Comparative Examples 1 to 6)

Peptide-conjugated (meth)acrylic copolymers having the configurations shown in Tables 1 to 3 were obtained in the same manner as in Example 1, except that the kind and blending amount of the (meth)acrylate compound were changed. Cell culture containers were obtained in the same manner as in Example 1, except that the obtained peptide-conjugated (meth)acrylic copolymer was used. Light acrylate (MTG-A manufactured by Kyoeisha Chemical Co., Ltd., methoxytriethylene glycol acrylate) was used for compounding in Comparative Examples 1 and 3, and Synthemax vitronectin substrate (manufactured by Corning Incorporated) was used instead of the peptide-conjugated (meth)acrylic copolymer in Comparative Example 7.

(Evaluation)

(1) Number average molecular weight of peptide-conjugated (meth)acrylic copolymer

The number average molecular weight of the obtained peptide-conjugated (meth)acrylic copolymer was measured by the above-described method.

(2) Retention time of peak top (condition 1)

HPLC measurement under the condition 1 was performed as follows, and the retention time of the peak top in the main peak having the largest area among signals to be detected was determined.

A measurement sample was obtained by adjusting the concentration of the peptide-conjugated (meth)acrylic copolymer (resin film composed of the scaffold material) to 2 mg/mL using a mixed solution of a 0.1 wt% aqueous formic acid solution and isopropyl alcohol (0.1 wt% aqueous formic acid solution/isopropyl alcohol = 7/3 [volume ratio]) as a solvent.

As a measuring instrument, an HPLC apparatus (High pressure gradient HPLC system Prominence manufactured by SHIMADZU CORPORATION), an HPLC column (XBridge (registered trademark) C18 [inner diameter 3.0 mm × length 150 mm, filler particle size 3.5 μm] manufactured by Waters Corporation), and an evaporative light scattering detector (ELSD LTII manufactured by SHIMADZU CORPORATION) were used. The analysis was performed by the following procedure.

A 0.1 wt% aqueous formic acid solution was used as the mobile phase A (liquid A), and isopropyl alcohol was used as the mobile phase B (liquid B). At the start of the measurement, the inside of the HPLC apparatus was filled with

a mixed solvent in which the mobile phase A/the mobile phase B was 7/3 in terms of volume ratio. In this state, the measurement sample (injection amount: 20 uL) was injected. Over 15 minutes immediately after the sample injection, the ratio of the mobile phase B was increased at a constant speed so that the ratio of the mobile phase B in the mobile phases was 100% in terms of volume ratio. After 15 minutes (at this point, the mobile phase was completely replaced by the mobile phase B), the mobile phase B was caused to flow for 15 minutes. The column temperature was set to 40°C, and the liquid delivery flow rate was set to a total flow rate of 0.3 mL/min.

As a nebulizer gas of the evaporative light scattering detector, nitrogen gas was used. The gas supply pressure was set to 350 kPa, and the drift tube temperature was set to 40°C. The determination of the baseline was performed by analyzing a blank test solution prepared in the same manner as in the preparation of the analysis sample except that the scaffold material was dissolved.

(3) Retention time of peak top (condition 2)

HPLC measurement under the condition 2 was performed in the same manner as in the condition 1 except for the following points, and the retention time of the peak top in the main peak having the largest area among signals to be detected was determined.

A measurement sample was obtained by adjusting the concentration of the peptide-conjugated (meth)acrylic copolymer (resin film composed of the scaffold material) to 2 mg/mL using a mixed solution of THF and IPA (THF : IPA = 7 : 3 (volume ratio)) as a solvent. The analysis was performed by the following procedure.

As the mobile phase A (liquid A), 0.1 wt/vol methanol formate was used, and as the mobile phase B (liquid B), a mixed solution of THF and isopropyl alcohol (THF : IPA = 7 : 3 [volume ratio]) was used. At the start of the measurement, the volume ratio of the mobile phase A was set to 100%, and the mobile phase A was caused to flow at a constant volume ratio of 100% for 0 minutes to 2 minutes. From 2 minutes to 5 minutes, the ratio of the mobile phase B was increased at a constant speed so that the mobile phase A/the mobile phase B was 90%/10% in terms of volume ratio at the time point of 5 minutes. From 5 minutes to 17 minutes, the ratio of the mobile phase B was increased at a constant speed so that the mobile phase B was 100% in terms of volume ratio at the time point of 17 minutes. From 17 minutes to 30 minutes, the mobile phase B was caused to flow at a constant volume ratio of 100%.

(4) Culture stability of cells (long-term culture stability)

The following liquid medium was prepared.

R-STEM (manufactured by ROHTO Pharmaceutical Co., Ltd.)

To the obtained cell culture container, 1 mL of phosphate buffered saline was added, the mixture was left to stand still in an incubator at 37°C for 1 hour, and then the phosphate buffered saline was removed from the cell culture container.

A cell suspension containing $5\times10^4$ cells (human fat-derived mesenchymal stem cell manufactured by LONZA KK., Model No.: PT-5006) was prepared in 1.5 mL of the liquid medium. This cell suspension was seeded into each well of a 6-well plate. Next, the 6-well plate was shaken 5 times to the right and left, and placed in an incubator at 37°C and a $CO_2$ concentration of 5% to perform culture.

The number of cells at 24 hours, 48 hours, and 72 hours after seeding of the cells was counted using Nucleo-Counter NC-3000 (manufactured by M&S TechnoSystems Inc.). When the doubling time of the cells between 24 hours and 48 hours after seeding was defined as T1, and the doubling time of the cells between 48 hours and 72 hours after seeding was defined as T2, T1 and T2 were calculated by the following formulas, respectively.

$$T1 = 24 \log 2 / (\log (N (48) / N (24))$$

$$T2 = 24 \log 2 / (\log (N (72) / N (48))$$

T1: Doubling time of cells from 24 hours to 48 hours after seeding
T2: Doubling time of cells from 48 hours to 72 hours after seeding
N (24): Cell number (cells) after 24 hours from seeding
N (48): Cell number (cells) after 48 hours from seeding
N (72): Cell number (cells) after 72 hours from seeding

From the doubling time (T1) and the doubling time (T2), a ratio (T1/T2) of T1 to T2 was calculated. The culture stability of cells was evaluated according to the following criteria. A larger ratio (T1/T2) means that the proliferation speed of cells is not decreased.

<Criteria for determining culture stability of cells (long-term culture stability)>

○○: The ratio (T1/T2) is 0.9 or more.
○: The ratio (T1/T2) is 0.7 or more and less than 0.9.
×: The ratio (T1/T2) is less than 0.7.

Fig. 3 is phase-contrast micrographs at 24 hours, 48 hours, and 72 hours after seeding of cells when cell culture is performed using scaffold materials for cell culture obtained in Examples 1 and 8 and Comparative Example 2. The ratio (T1/T2) was 0.85 in Example 1, the ratio (T1/T2) was 0.99 in Example 8, and the ratio (T1/T2) was 0.5 in Comparative Example 2.

(5) Solubility in ethanol

The degree of solubility of the obtained scaffold material in ethanol was measured by the following method. The scaffold material was mixed with ethanol and stirred at 60°C, and after 30 minutes, the presence or absence of the undissolved scaffold material and the transparency of the solution were visually checked. The maximum solution concentration at which there was no undissolved scaffold material and the solution became clear was defined as the degree of solubility. The solubility in ethanol was evaluated according to the following criteria.

<Criteria for determining solubility in ethanol>

○○: The degree of solubility is 1 wt% or more.
○: The degree of solubility is 0.1 wt% or more and less than 1 wt%.
×: The degree of solubility is less than 0.1 wt%.

Compositions and results are shown in Tables 1 to 3 below.

[Table 1]

| | | | | Example 1 | Example 2 | Example 3 | Example 4 | Example 5 | Example 6 |
|---|---|---|---|---|---|---|---|---|---|
| (Meth)acrylic copolymer moiety | (Meth)acrylate compound (A) | Ethyl acrylate | mol% | | | | | | |
| | | Butyl acrylate | mol% | 25 | 53 | | | | |
| | | Butyl methacrylate | mol% | | | 25 | 54 | | |
| | | Octyl acrylate | mol% | | | | | 13 | |
| | | Dodecyl acrylate | mol% | | | | | | 10 |
| | | Hydroxyethyl methacrylate | mol% | | | | | | |
| | | MTG-A | mol% | | | | | | |
| | (Meth) acrylate compound (B) | Acrylic acid | mol% | 70 | 42 | 70 | 41 | 82 | 95 |
| | | Methacrylic acid | mol% | | | | | | |
| Content ratio of structural unit derived from (meth)acrylate compound (A) in 100 mol% of total structural units of (meth)acrylic copolymer moiety | | | mol% | 26.3 | 55.8 | 26.3 | 56.8 | 13.7 | 10.5 |
| Peptide moiety | c-RGDfK | | mol% | 5 | 5 | 5 | 5 | 5 | 5 |
| Number average molecular weight of peptide-conjugated (meth)acrylic copolymer | | | | 50000 | 50000 | 50000 | 50000 | 50000 | 50000 |
| Retention time of peak top | Condition 1 | | min | 23 | 28 | 25 | 29 | 25 | 29 |
| | Condition 2 | | min | 2 | 2 | 2 | 2 | 2 | 2 |
| Culture stability of cells (long-term culture stability) | | | | ○ | ○○ | ○ | ○○ | ○ | ○ |
| Solubility in ethanol | | | | ○○ | ○ | ○○ | ○ | ○○ | ○ |

[Table 2]

| | | | | Example 7 | Example 8 | Example 9 | Example 10 | Comparative Example 1 | Comparative Example 2 |
|---|---|---|---|---|---|---|---|---|---|
| (Meth)acrylic copolymer moiety | (Meth)acrylate compound (A) | Ethyl acrylate | mol% | | | | | | |
| | | Butyl acrylate | mol% | | | | | | |
| | | Butyl methacrylate | mol% | | | | 11 | | 20.3 |
| | | Octyl acrylate | mol% | | | 4 | | | |
| | | Dodecyl acrylate | mol% | 22 | 50 | | | | |
| | | Hydroxyethyl methacrylate | mol% | | | | | | 72 |
| | | MTG-A | mol% | | | | | 55 | |
| | (Meth)acrylate compound (B) | Acrylic acid | mol% | 73 | 45 | 91 | 84 | 40 | |
| | | Methacrylic acid | mol% | | | | | | 2.7 |
| Content ratio of structural unit derived from (meth) acrylate compound (A) in 100 mol% of total structural units of (meth) acrylic copolymer moiety | | | mol% | 23.2 | 52.6 | 4.2 | 11.6 | 0 | 21.4 |
| Peptide moiety | c-RGDfK | | mol% | 5 | 5 | 5 | 5 | 5 | 5 |
| Number average molecular weight of peptide-conjugated (meth)acrylic copolymer | | | | 50000 | 50000 | 50000 | 50000 | 50000 | 50000 |
| Retention time of peak top | Condition 1 | | min | > 30 | > 30 | 5 | 5 | 3 | 3 |
| | Condition 2 | | min | 21 | 24 | 2 | 2 | 2 | 2 |
| Culture stability of cells (long-term culture stability) | | | | ○○ | ○○ | ○ | ○ | × | × |
| Solubility in ethanol | | | | ○ | ○○ | ○○ | ○○ | ○○ | ○ |

[Table 3]

| | | | | Comparative Example 3 | Comparative Example 4 | Comparative Example 5 | Comparative Example 6 | Comparative Example 7 |
|---|---|---|---|---|---|---|---|---|
| (Meth)acrylic copolymer moiety | (Meth)acrylate compound (A) | Ethyl acrylate | mol% | | 20 | | | |
| | | Butyl acrylate | mol% | | | 5 | | |
| | | Butyl methacrylate | mol% | | | | 5 | |
| | | Octyl acrylate | mol% | | | | | |
| | | Dodecyl acrylate | mol% | | | | | |
| | | Hydroxyethyl methacrylate | mol% | | | | | |
| | | MTG-A | mol% | 1.5 | | | | |
| | (Meth)acrylate compound (B) | Acrylic acid | mol% | | 75 | 90 | 90 | |
| | | Methacrylic acid | mol% | 93.5 | | | | |
| Content ratio of structural unit derived from (meth)acrylate compound (A) in 100 mol% of total structural units of (meth)acrylic copolymer moiety | | | mol% | 0 | 21 | 5.3 | 5.3 | |
| Peptide moiety | c-RGDfK | | mol% | 5 | 5 | 5 | 5 | |
| Number average molecular weight of peptide-conjugated (meth) acrylic copolymer | | | | 50000 | 50000 | 50000 | 50000 | |
| Retention time of peak top | Condition 1 | | min | 3 | 3 | 3 | 2 | 2 |
| | Condition 2 | | min | 2 | 2 | 2 | 2 | 2 |
| Culture stability of cells (long-term culture stability) | | | | × | × | × | × | × |
| Solubility in ethanol | | | | × | ○ | × | × | ○ |

## EXPLANATION OF SYMBOLS

1: Cell culture container
2: Container body
2a: Surface
3: Resin film
5: Cell culture microcarrier
6: Base material particle
7: Coating layer

## Claims

1. A scaffold material for cell culture, comprising:

   a peptide-conjugated (meth)acrylic copolymer having a (meth)acrylic copolymer moiety and a peptide moiety bonded to the (meth)acrylic copolymer moiety,
   when the peptide-conjugated (meth)acrylic copolymer is subjected to high performance liquid chromatography measurement under the following condition 1, a peak top of a main peak having the largest area among signals being not detected within a retention time of 4 minutes:
   the condition 1 being:

   a column of C18 (Inner diameter 3.0 mm × length 150 mm, filler particle size 3.5 μm),
   a column temperature of 40°C,
   a flow rate of 0.3 mL/min,
   a detector of an evaporative light scattering detector,
   a mobile phase A (liquid A) of a 0.1 wt% aqueous formic acid solution,
   a mobile phase B (liquid B) of isopropyl alcohol,
   a retention time of 0 minutes, in which the liquid A/the liquid B (volume ratio) is 70%/30%,
   a retention time of 0 minutes to 15 minutes, in which the liquid A/the liquid B (volume ratio) is changed from 70%/30% to 0%/100%, and
   a retention time of 15 minutes to 30 minutes, in which the liquid A/the liquid B (volume ratio) - 0%/100% is maintained.

2. The scaffold material for cell culture according to claim 1, wherein, when the peptide-conjugated (meth)acrylic copolymer is subjected to high performance liquid chromatography measurement under the condition 1, a retention time of a peak top in a main peak having the largest area among signals to be detected is 5 minutes or more and 30 minutes or less.

3. A scaffold material for cell culture, comprising:

   a peptide-conjugated (meth)acrylic copolymer having a (meth)acrylic copolymer moiety and a peptide moiety bonded to the (meth)acrylic copolymer moiety,
   when the peptide-conjugated (meth)acrylic copolymer is subjected to high performance liquid chromatography measurement under the following condition 2, a retention time of a peak top in a main peak having the largest area among signals to be detected being 5 minutes or more and 30 minutes or less:
   the condition 2 being:

   a column of C18 (Inner diameter 3.0 mm × length 150 mm, filler particle size 3.5 μm),
   a column temperature of 40°C,
   a flow rate of 0.3 mL/min,
   a detector of an evaporative light scattering detector,
   a mobile phase A (liquid A) of 0.1 wt/vol methanol formate,
   a mobile phase B (liquid B) of THF/isopropyl alcohol = 7/3 (volume ratio),
   a retention time of 0 minutes to 2 minutes, in which the liquid A/the liquid B (volume ratio) = 100%/0% is maintained,

a retention time of 2 minutes to 5 minutes, in which the liquid A/the liquid B (volume ratio) is changed from 100%/0% to 90%/10%,
a retention time of 5 minutes to 17 minutes, in which the liquid A/the liquid B (volume ratio) is changed from 90%/10% to 0%/100%, and
a retention time of 17 minutes to 30 minutes, in which the liquid A/the liquid B (volume ratio) - 0%/100% is maintained.

4. The scaffold material for cell culture according to any one of claims 1 to 3, wherein the (meth)acrylic copolymer moiety has a structural unit derived from a (meth)acrylate compound (A) represented by the following Formula (A1) or the following Formula (A2):

[Chemical 1]

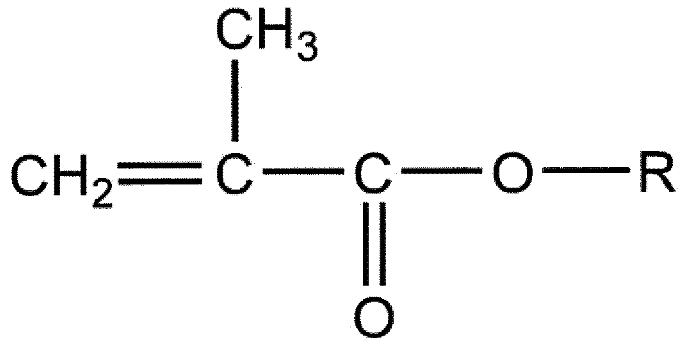

···(A1)

wherein R represents a hydrocarbon group having 2 or more and 18 or less carbon atoms,

[Chemical 2]

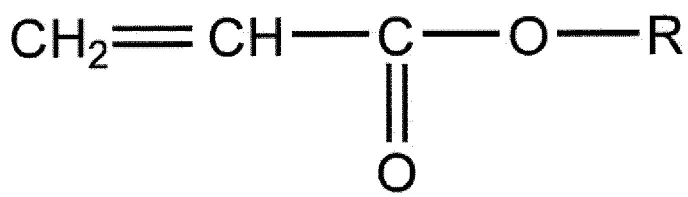

···(A2)

wherein R represents a hydrocarbon group having 2 or more and 18 or less carbon atoms.

5. The scaffold material for cell culture according to any one of claims 1 to 4, wherein

the (meth)acrylic copolymer moiety has a structural unit derived from a (meth)acrylate compound (B) having a functional group capable of reacting with an amino group or a carboxyl group, and
in the peptide-conjugated (meth)acrylic copolymer, the peptide moiety is bonded to the functional group capable of reacting with an amino group or a carboxyl group.

6. The scaffold material for cell culture according to any one of claims 1 to 5, wherein a content ratio of the structural unit derived from the (meth)acrylate compound (A) is 25 mol% or more and 98 mol% or less in 100 mol% of the total structural units of the (meth)acrylic copolymer moiety.

7. The scaffold material for cell culture according to any one of claims 1 to 6, wherein a number average molecular weight of the peptide-conjugated (meth)acrylic copolymer is 5000 or more.

8. The scaffold material for cell culture according to any one of claims 1 to 7, wherein in the peptide-conjugated (meth)acrylic copolymer, a content ratio of the peptide moiety is 0.5 mol% or more and 25 mol% or less.

9. The scaffold material for cell culture according to any one of claims 1 to 8, wherein the peptide moiety has an RGD sequence.

[FIG. 1]

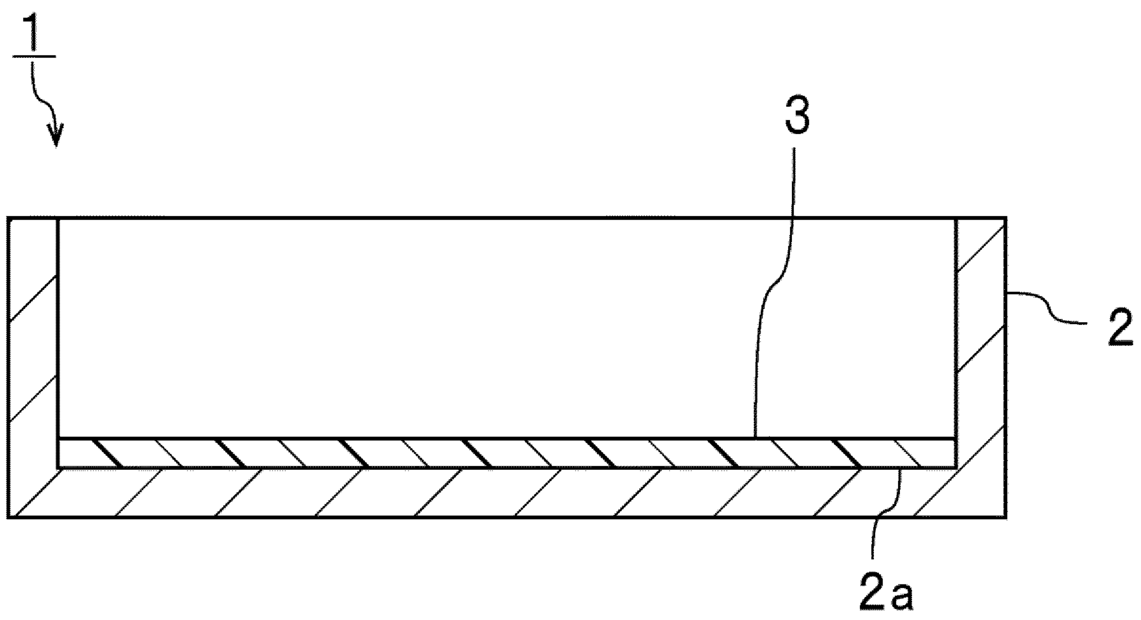


[FIG. 2]

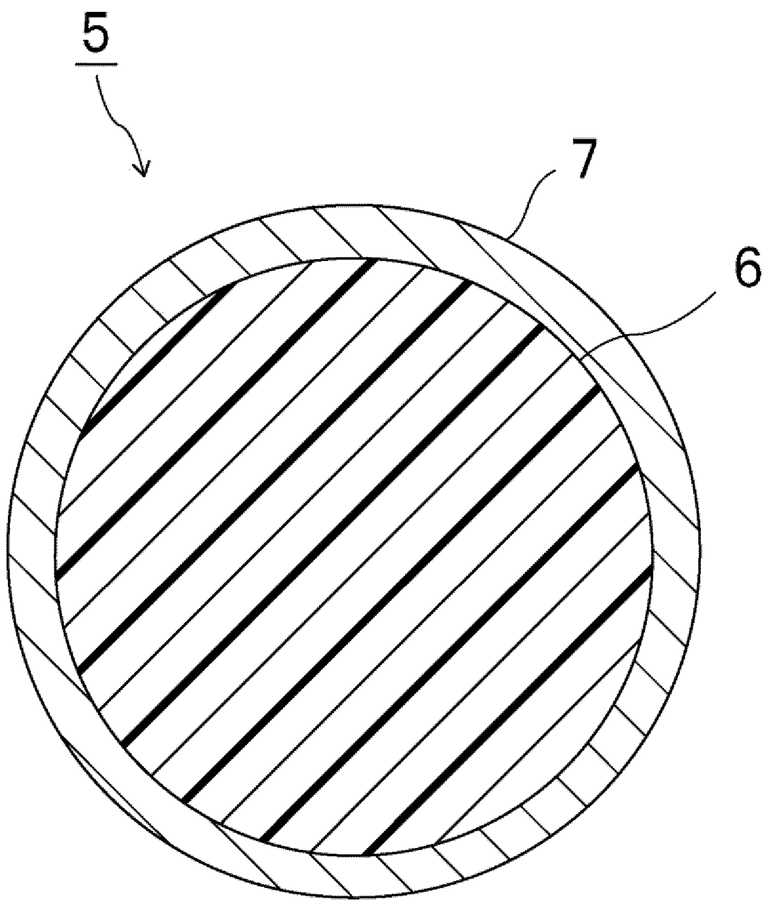

[FIG. 3]

| | Ratio (T1/T2) | 24 hours after seeding | 48 hours after seeding | 72 hours after seeding |
|---|---|---|---|---|
| Example 1 | 0.85 | 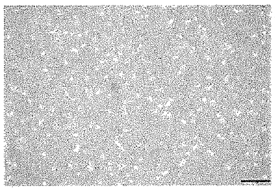 | 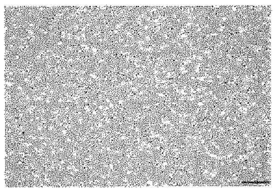 | 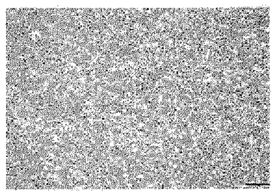 |
| Example 8 | 0.99 | 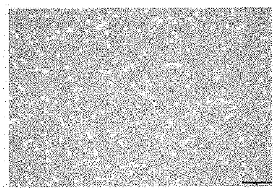 | 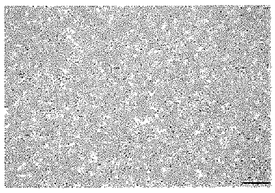 | 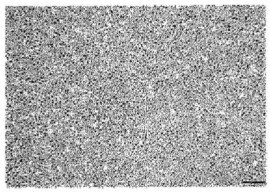 |
| Comparative Example 2 | 0.5 | 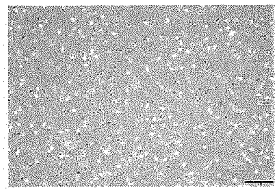 | 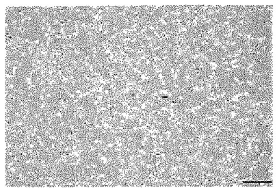 | 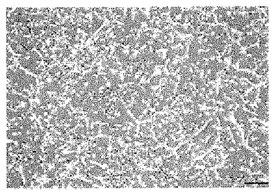 |

## INTERNATIONAL SEARCH REPORT

International application No.
**PCT/JP2022/047836**

**A. CLASSIFICATION OF SUBJECT MATTER**

***C12M 3/00***(2006.01)i; ***C07K 5/09***(2006.01)i; ***C07K 5/10***(2006.01)i; ***C07K 5/11***(2006.01)i; ***C07K 7/06***(2006.01)i; ***C07K 17/08***(2006.01)i; ***C08F 261/12***(2006.01)i; ***C08G 81/02***(2006.01)i; ***C08L 101/06***(2006.01)i; ***C12M 1/00***(2006.01)i; ***C12N 1/00***(2006.01)i; ***C12N 5/071***(2010.01)i

FI: C12M3/00 A ZNA; C12N1/00 A; C07K5/10; C07K7/06; C12N5/071; C12M1/00 A ZNA; C08L101/06; C08F261/12; C07K17/08; C07K5/09; C07K5/11; C08G81/02; C12M1/00 C ZNA

According to International Patent Classification (IPC) or to both national classification and IPC

**B. FIELDS SEARCHED**

Minimum documentation searched (classification system followed by classification symbols)

C12M3/00; C07K5/09; C07K5/10; C07K5/11; C07K7/06; C07K17/08; C08F261/12; C08G81/02; C08L101/06; C12M1/00; C12N1/00; C12N5/071

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN); JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2021-23287 A (SEKISUI CHEMICAL CO LTD) 22 February 2021 (2021-02-22) claims 1, 8, paragraphs [0027], [0028], [0040], [0044], [0047], [0049], [0052], [0080]-[0082], [0089], [0091], [0092], [0097] | 1-9 |
| Y | JP 2021-3061 A (SEKISUI CHEMICAL CO LTD) 14 January 2021 (2021-01-14) claims 1, 3, 8, paragraphs [0026], [0033] | 1-9 |
| A | WO 2020/241675 A1 (SEKISUI CHEMICAL CO LTD) 03 December 2020 (2020-12-03) entire description, in particular, comparative examples 3, 4 | 1-9 |
| P, X | WO 2022/168872 A1 (SEKISUI CHEMICAL CO LTD) 11 August 2022 (2022-08-11) entire description | 1-9 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2023** | **21 February 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/047836**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

"The form of Annex C/ST.25 text file" above shall read as "the form of ST.26.".

Form PCT/ISA/210 (continuation of first sheet) (January 2015)

**INTERNATIONAL SEARCH REPORT**
**Information on patent family members**

International application No.
**PCT/JP2022/047836**

| Patent document cited in search report | Publication date (day/month/year) | Patent family member(s) | Publication date (day/month/year) |
|---|---|---|---|
| JP 2021-23287 A | 22 February 2021 | (Family: none) | |
| JP 2021-3061 A | 14 January 2021 | (Family: none) | |
| WO 2020/241675 A1 | 03 December 2020 | US 2022/0228127 A1<br>entire description, in particular, comparative examples 3, 4 | |
| WO 2022/168872 A1 | 11 August 2022 | WO 2022/168871 A1<br>WO 2022/168870 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012158235 A2
- JP H5292957 A


### Non-patent literature cited in the description


- *The Journal of Cell Biology,* September 1995, vol. 130 (5), 1189-1196
- *PROTEIN, NUCLEIC ACID, AND ENZYME,* 2000, vol. 45 (15), 2477
- *Pathophysiology,* 1990, vol. 9 (7), 527-535
- *Osaka Medical Center and Research Institute for Maternal and Child Health magazine,* 1992, vol. 8 (1), 58-66